# FASCICULE DE BREVET EUROPEEN

(11) **EP 1 473 023 B1**
(45) Date de publication et mention de la délivrance du brevet: **03.01.2007**
(21) Numéro de dépôt: 04291085.1
(22) Date de dépôt: 27.04.2004
(51) Int. Cl.: A61K 8/41, A61Q 5/10

(54) **Composition tinctoriale comprenant le 2-chloro 6-méthyl 3-amino phénol à titre de coupleur, le para-aminophénol et le 3-méthyl 4-amino phénol à titre de base d'oxydation et un polymère épaississant associatif**
Färbemischung, die 2-Chlor 6-Methyl 3-Amino Phenol als Kuppler, para-Aminophenol und 3-Methyl 4-Amino Phenol als Entwickler und ein assoziatives polymeren Verdickungsmittel enthält
Dyeing composition comprising 2-chloro 6-methyl 3-amino phenol as a coupling agent, para-aminophenol and 3-methyl 4-amino phenol as developper and a thickening associative polymer

(30) Priorité: 29.04.2003 FR 0305243
(43) Date de publication de la demande: 03.11.2004
(73) Titulaire: L'ORÉAL, 75008 Paris (FR)
(72) Inventeur: Audousset, Marie-Pascale, 92600 Asnieres (FR)
(74) Mandataire: Fevrier, Murielle Françoise E.

(56) Documents cités:
- WO-A-02/076923
- WO-A-02/096382
- DE-U- 20 107 481
- US-B1- 6 503 282

## Description

L'invention a pour objet une composition pour la teinture d'oxydation des fibres kératiniques, et en particulier des fibres kératiniques humaines telles que les cheveux, comprenant le 2-chloro 6-méthyl 3-amino phénol à titre de coupleur, le para-aminophénol à titre de première base d'oxydation, le 3-méthyl 4-amino phénol à titre de deuxième base d'oxydation et un polymère épaississant associatif.

Il est connu de teindre les fibres kératiniques, et en particulier les fibres kératiniques humaines telles que les cheveux, avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il a déjà été proposé, notamment dans la demande de brevet allemand DE 30 16 008 des compositions pour la teinture d'oxydation des fibres kératiniques comprenant le 2-chloro 6-méthyl 3-amino phénol ou le 2-méthyl 6-chloro 3-amino phénol à titre de coupleur, en association avec des bases d'oxydation classiquement utilisées pour la teinture d'oxydation, telles que par exemple certaines para-phénylènediamines ou du para-aminophénol.

La demande de brevet WO 96/15766 décrit des compositions pour la teinture d'oxydation des fibres kératiniques comprenant l'association spécifique du 2-chloro 6-méthyl 3-amino phénol à titre de coupleur et d'une base d'oxydation particulière choisie parmi les dérivés de para-aminophénol substitué en position 2 ou 3 comme par exemple le 3-méthyl 4-amino phénol, le 2-allyl 4-amino phénol ou bien encore le 2-aminométhyl 4-amino phénol.

Il a aussi été proposé, dans la demande de brevet EP 0 966 251, des compositions pour la teinture d'oxydation des fibres kératiniques contenant le 2-chloro 6-méthyl 3-amino phénol à titre de coupleur associé à au moins deux bases d'oxydation de nature différente choisies parmi les para-phénylènediamines, les bases doubles, les para-aminophénols, les ortho-aminophénols et les bases d'oxydation hétérocycliques.

De telles compositions ne sont cependant pas entièrement satisfaisantes notamment du point de vue de la tenue des colorations obtenues vis-à-vis des diverses agressions que peuvent subir les cheveux, et en particulier vis-à-vis des shampooings, de la lumière, de la sueur et des déformations permanentes et du point de vue de la puissance des colorations obtenues.

Le document brevet WO-A-02/096382 décrit une composition pour la teinture d'oxydation des fibres kératiniques comprenant, dans un milieu de teinture approprié :
- le coupleur 2-chloro 6-méthyl 3-amino phénol
- la première base d'oxydation para-aminophénol
- la deuxième base d'oxydation 3-méthyl 4-amino phénol, et
- le polymère Polyquaternium-2.

Le but de la présente invention est de fournir de nouvelles compositions pour la teinture d'oxydation des fibres kératiniques ne présentant pas les inconvénients de celles de l'art antérieur. En particulier, le but de la présente invention est de fournir de nouvelles compositions conduisant à des colorations aux nuances variées, chromatiques, puissantes, esthétiques, peu sélectives et résistant bien aux diverses agressions que peuvent subir les cheveux tels que les shampooings, la lumière, la sueur et les déformations permanentes.

Ce but est atteint avec la présente invention qui a pour objet une composition pour la teinture d'oxydation des fibres kératiniques comprenant, dans un milieu de teinture approprié :
- au moins un coupleur choisi parmi le 2-chloro 6-méthyl 3-amino phénol et ses sels d'addition ;
- au moins une première base d'oxydation choisie parmi le para-aminophénol et ses sels d'addition ;
- au moins une deuxième base d'oxydation choisie parmi le 3-méthyl 4-amino phénol et ses sels d'addition ; et
- au moins un polymère épaississant associatif.

La composition de la présente invention permet en particulier d'obtenir une coloration des fibres kératiniques chromatique, très puissante, peu sélective et tenace.

L'invention a aussi pour objet un procédé de teinture d'oxydation des fibres kératiniques, et en particulier des fibres kératiniques humaines telles que les cheveux, mettant en oeuvre cette composition.

Un autre objet de l'invention est l'utilisation de la composition de la présente invention pour la teinture d'oxydation des fibres kératiniques, et en particulier des fibres kératiniques humaines telles que les cheveux.

Les polymères associatifs utilisables selon l'invention sont des polymères hydrosolubles ou hydrodispersibles capables, dans un milieu aqueux, de s'associer réversiblement entre eux ou avec d'autres molécules. Leur structure chimique comprend des zones hydrophiles et des zones hydrophobes caractérisées par au moins une chaîne grasse en C₈-C₃₀ et de préférence en C₁₀-C₃₀.

Ces polymères sont issus soit d'une polymérisation radicalaire ou d'une polycondensation à partir d'au moins un monomère dont l'un au moins possède une chaîne grasse en C₈-C₃₀, de préférence en C₁₀-C₃₀, soit du greffage sur un polymère, de préférence polyhydroxylé, d'un composé comportant au moins une chaîne grasse en C₈-C₃₀, de préférence en C₁₀-C₃₀.

On entend par "polymère épaississant" un polymère qui présente avantageusement en solution ou en dispersion, à 5 % de matière active dans l'eau, une viscosité mesurée au moyen du rhéomètre Rhéomat RM 180, à 25 °C, au moins égale à 500 cP, à un taux de cisaillement de 100 s⁻¹.

Les polymères associatifs selon l'invention peuvent être anioniques, cationiques, amphotères ou non ioniques. De préférence, les polymères associatifs sont anioniques, cationiques ou non ioniques.

Parmi les polymères associatifs de type anioniques, on peut citer :
- (1) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux-ci, et dont le motif éther d'allyle à chaîne grasse correspond au monomère de formule (V) suivante :

   CH₂ = C R' CH₂ O Bₙ R (V)

   dans laquelle :
   - R' désigne H ou CH₃ ;
   - B désigne le radical éthylèneoxy ;
   - n est nul ou désigne un entier allant de 1 à 100 ;
   - R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone.

   Un motif de formule (V) plus particulièrement préféré est un motif dans lequel R' désigne H, n est égal à 10 et R désigne un radical stéaryle (C₁₈).
   Des polymères associatifs anioniques de ce type sont décrits et préparés, selon un procédé de polymérisation en émulsion, dans le brevet EP-0 216479.
   Parmi ces polymères associatifs anioniques, on préfère particulièrement selon l'invention les polymères formés à partir de 20 à 60 % en poids d'acide acrylique et / ou d'acide méthacrylique, de 5 à 60 % en poids de (méth)acrylates d'alkyles inférieurs, de 2 à 50 % en poids d'éther d'allyle à chaîne grasse de formule (V) et de 0 à 1 % en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 ® et SALCARE SC90 ® qui sont des émulsions aqueuses à 30 % d'un terpolymère réticulé d'acide méthacrylique, d'acrylate d'éthyle et de steareth-10-allyl éther (40 / 50 / 10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (VI) suivante : 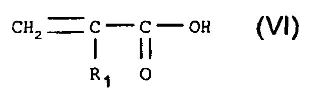 dans laquelle R₁ désigne H ou CH₃ ou C₂H₅, c'est-à-dire des motifs acide acrylique, acide méthacrylique ou acide éthacrylique, et dont le motif hydrophobe de type ester d'alkyle (C₁₀-C₃₀) d'acide carboxylique insaturé correspond au monomère de formule (VII) suivante 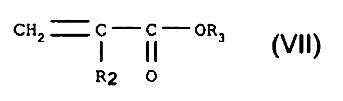 dans laquelle :
   - R₂ désigne H ou CH₃ ou C₂H₅, c'est-à-dire des motifs acrylates, méthacrylates ou éthacrylates, et de préférence H (motifs acrylates) ou CH₃ (motifs méthacrylates) ;
   - R₃ désignant un radical alkyle en C₁₀-C₃₀, et de préférence en C₁₂-C₂₂.

   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US-3 915 921 et 4 509 949.
   Parmi ce type de polymères associatifs anioniques, on utilisera plus particulièrement des polymères formés à partir d'un mélange de monomères comprenant :
   (i) essentiellement de l'acide acrylique ;
   (ii) un ester de formule (VII) décrite ci-dessus et dans laquelle R₂ désigne H ou CH₃, R₃ désignant un radical alkyle ayant de 12 à 22 atomes de carbone ;
   (iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

   Parmi ce type de polymères associatifs anioniques, on utilisera plus particulièrement ceux constitués de 95 à 60 % en poids d'acide acrylique (motif hydrophile), de 4 à 40 % en poids d'acrylate d'alkyle en C₁₀-C₃₀ (motif hydrophobe), et de 0 à 6 % en poids de monomère polymérisable réticulant, ou bien ceux constitués de 98 à 96 % en poids d'acide acrylique (motif hydrophile), de 1 à 4 % en poids d'acrylate d'alkyle en C₁₀-C₃₀ (motif hydrophobe), et de 0,1 à 0,6 % en poids de monomère polymérisable réticulant tel que ceux décrits précédemment.
   Parmi lesdits polymères ci-dessus, on préfère tout particulièrement selon la présente invention, les produits vendus par la société GOODRICH sous les dénominations commerciales PEMULEN TR1 ®, PEMULEN TR2 ®, CARBOPOL 1382 ®, et encore plus préférentiellement le PEMULEN TR1 ®, et le produit vendu par la société S.E.P.P.I.C. sous la dénomination COATEX SX ®.
- (III) les terpolymères d'anhydride maléique / α-oléfine en C₃₀-C₃₈ / maléate d'alkyle tel que le produit (copolymère anhydride maléique / α-oléfine en C₃₀-C₃₈ / maléate d'isopropyle) vendu sous le nom PERFORMA V 1608 ® par la société NEWPHASE TECHNOLOGIES.
- (IV) les terpolymères acryliques comprenant :
   (a) environ 20 % à 70 % en poids d'un acide carboxylique à insaturation α,β-monoéthylénique ;
   (b) environ 20 à 80 % en poids d'un monomère à insaturation α,β-monoéthylénique non-tensio-actif différent de (a) ;
   (c) environ 0,5 à 60 % en poids d'un mono-uréthane non-ionique qui est le produit de réaction d'un tensio-actif monohydrique avec un monoisocyanate à insaturation monoéthylénique ;
   tels que ceux décrits dans la demande de brevet EP-A-0 173 109 et plus particulièrement celui décrit dans l'exemple 3, à savoir, un terpolymère acide méthacrylique / acrylate de méthyle / diméthyl métaisopropényl benzyl isocyanate d'alcool béhényle éthoxylé (40 OE) en dispersion aqueuse à 25 %.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.
   Préférentiellement, ces composés comprennent également comme monomère un ester d'acide carboxylique à insaturation α,β-monoéthylénique et d'alcool en C₁-C₄.
   A titre d'exemple de ce type de composé, on peut citer l'ACULYN 22 ® vendu par la société ROHM et HAAS, qui est un terpolymère acide méthacrylique / acrylate d'éthyle / méthacrylate de stéaryle oxyalkyléné.
- (VI) les polyuréthanes associatifs à caractère anionique tels que le VISCOPHOBE DB 1000 de la société UNION CARBIDE.

Parmi les polymères associatifs de type cationique, on peut citer parmi eux :
- (1) les polyuréthanes associatifs cationiques dont la famille a été décrite par la demanderesse dans la demande de brevet français FR 0 009 609. Elle peut être représentée par la formule générale (VIII) suivante :

   R-X-(P)ₙ-[L-(Y)ₘ]ᵣ-L'-(P')ₚ-X'-R' (VIII)

   dans laquelle :
   - R et R', identiques ou différents, représentent un groupement hydrophobe ou un atome d'hydrogène ;
   - X et X', identiques ou différents, représentent un groupement comportant une fonction amine portant ou non un groupement hydrophobe, ou encore le groupement L" ;
   - L, L' et L", identiques ou différents, représentent un groupement dérivé d'un diisocyanate ;
   - P et P', identiques ou différents, représentent un groupement comportant une fonction amine portant ou non un groupement hydrophobe ;
   - Y représente un groupement hydrophile ;
   - r est un nombre entier compris entre 1 et 100, de préférence entre 1 et 50 et en particulier entre 1 et 25 ;
   - n, m, et p valent chacun indépendamment des autres entre 0 et 1000 ; la molécule contenant au moins une fonction amine protonée ou quaternisée et au moins un groupement hydrophobe.

   Dans un mode de réalisation préféré de ces polyuréthanes, les seuls groupements hydrophobes sont les groupes R et R' aux extrémités de chaîne.
   Une famille préférée de polyuréthanes associatifs cationiques est celle correspondant à la formule (VIII) décrite ci-dessus et dans laquelle :
   - R et R' représentent tous les deux indépendamment un groupement hydrophobe ;
   - X et X' représentent chacun un groupe L" ;
   - n et p valent entre 1 et 1000 ; et
   - L, L', L", P, P', Y et m ont la signification indiquée ci-dessus.

   Une autre famille préférée de polyuréthanes associatifs cationiques est celle correspondant à la formule (VIII) ci-dessus dans laquelle :
   - R et R' représentent tous les deux indépendamment un groupement hydrophobe ;
   - X et X' représentent chacun un groupe L" ;
   - n et p valent 0 ; et
   - L, L', L", Y et m ont la signification indiquée ci-dessus.

   Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.
   Encore une autre famille préférée de polyuréthanes associatifs cationiques est celle correspondant à la formule (VIII) ci-dessus dans laquelle :
   - R et R' représentent tous les deux indépendamment un groupement hydrophobe ;
   - X et X' représentent tous les deux indépendamment un groupement comportant une amine quaternaire ;
   - n et p valent zéro ; et
   - L, L', Y et m ont la signification indiquée ci-dessus.

   La masse moléculaire moyenne en nombre des polyuréthanes associatifs cationiques est comprise de préférence entre 400 et 500 000, en particulier entre 1000 et 400 000 et idéalement entre 1000 et 300 000.
   Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
   Préférentiellement, le groupement hydrocarboné provient d'un composé monofonctionnel.
   A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.
   Lorsque X et / ou X' désignent un groupement comportant une amine tertiaire ou quaternaire, X et / ou X' peuvent représenter l'une des formules suivantes : 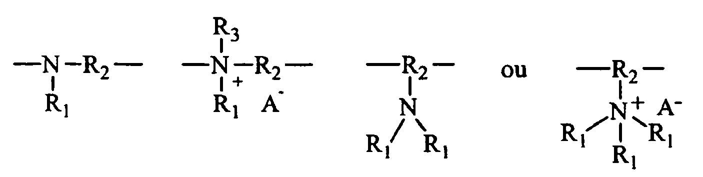 pour X 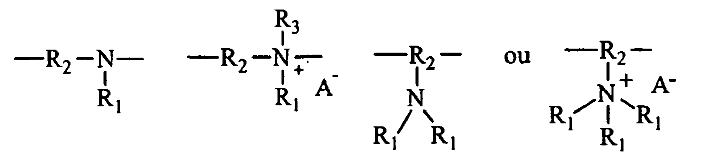 pour X'
   dans lesquelles :
   - R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
   - R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
   - A- est un contre-ion physiologiquement acceptable.

   Les groupements L, L' et L" représentent un groupe de formule : 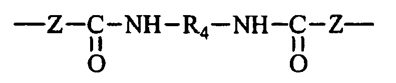 dans laquelle :
   - Z représente -O-, -S- ou -NH- ; et
   - R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

   Les groupements P et P', comprenant une fonction amine peuvent représenter au moins l'une des formules suivantes : 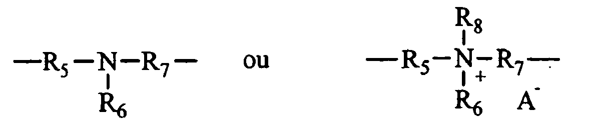 ou 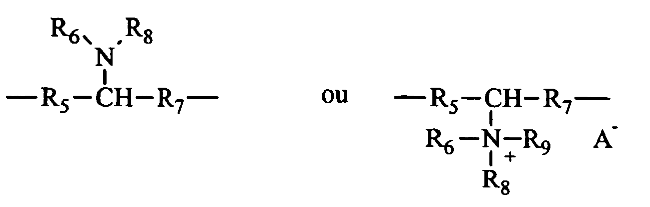 ou 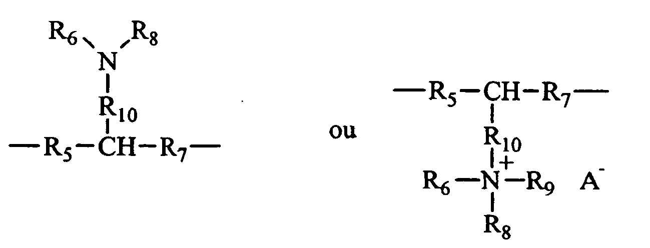 dans lesquelles :
   - R₅ et R₇ ont les mêmes significations que R₂ défini précédemment ;
   - R₆, R₈ et R₉ ont les mêmes significations que R₁ et R₃ définis précédemment ;
   - R₁₀ représente un groupe alkylène, linéaire ou ramifié, éventuellement insaturé et pouvant contenir un ou plusieurs hétéroatomes choisis parmi N, 0, S et P ; et
   - A- est un contre-ion physiologiquement acceptable.

   En ce qui concerne la signification de Y, on entend par groupement hydrophile, un groupement hydrosoluble polymérique ou non.
   A titre d'exemple, on peut citer, lorsqu'il ne s'agit pas de polymères, l'éthylèneglycol, le diéthylèneglycol et le propylèneglycol.
   Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).
   Les polyuréthanes associatifs cationiques de formule (VIII) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.
   Un premier type de composés entrant dans la préparation du polyuréthane de formule (VIII) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
   Comme indiqué précédemment, ce composé peut comporter plus d'un motif à fonction amine. Il s'agit alors d'un polymère portant une répétition du motif à fonction amine.
   Ce type de composés peut être représenté par l'une des formules suivantes :

   HZ-(P)ₙ-ZH,

   ou

   HZ-(P')ₚ-ZH

   dans lesquelles Z, P, P', n et p sont tels que définis plus haut.
   A titre d'exemple de composé à fonction amine, on peut citer la N-méthyldiéthanolamine ; la N-tert-butyl-diéthanolamine ; la N-sulfoéthyldiéthanolamine.
   Le deuxième composé entrant dans la préparation du polyuréthane de formule (VIII) est un diisocyanate correspondant à la formule :

   O=C=N-R₄-N=C=O

   dans laquelle R₄ est défini plus haut.
   A titre d'exemple, on peut citer le méthylènediphényl-diisocyanate ; le méthylènecyclohexanediisocyanate ; l'isophorone-diisocyanate ; le toluènediisocyanate ; le naphtalènediisocyanate ; le butanediisocyanate ; l'hexanediisocyanate.
   Un troisième composé entrant dans la préparation du polyuréthane de formule (VIII) est un composé hydrophobe destiné à former les groupes hydrophobes terminaux du polymère de formule (VIII).
   Ce composé est constitué d'un groupe hydrophobe et d'une fonction à hydrogène labile, par exemple une fonction hydroxyle, amine primaire ou secondaire, ou thiol.
   A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné -hydroxyle.
   Le groupe hydrophobe du polyuréthane de formule (VIII) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate, etc.
   Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.
   Les fonctions à hydrogène labile sont des fonctions alcool, amine primaire ou secondaire, ou thiol. Ce composé peut être un polymère terminé aux extrémités des chaînes par l'une de ces fonctions à hydrogène labile.
   A titre d'exemple, on peut citer, lorsqu'il ne s'agit pas de polymères, l'éthylèneglycol, le diéthylèneglycol et le propylèneglycol.
   Lorsqu'il s'agit d'un polymère hydrophile, on peut citer à titre d'exemple les polyéthers, les polyesters sulfonés, les polyamides sulfonés ou un mélange de ces polymères. A titre préférentiel, le composé hydrophile est un polyéther et notamment un poly(oxyde d'éthylène) ou poly(oxyde de propylène).
   Le groupe hydrophile noté Y dans la formule (VIII) est facultatif. En effet, les motifs à fonction amine quaternaire ou protonée peuvent suffire à apporter la solubilité ou l'hydrodispersibilité nécessaire pour ce type de polymère dans une solution aqueuse.
   Bien que la présence d'un groupe Y hydrophile soit facultative, on préfère cependant des polyuréthanes associatifs cationiques comportant un tel groupe.
- (II) les dérivés de cellulose quaternisée et les polyacrylates à groupements latéraux aminés non cycliques.
   Les dérivés de cellulose quaternisée sont en particulier,
   - les celluloses quaternisées modifiées par des groupements comportant au moins une chaîne grasse, tels que les groupes alkyle, arylalkyle, alkylaryle comportant au moins 8 atomes de carbone ou des mélanges de ceux-ci,
   - les hydroxyéthylcelluloses quaternisées modifiées par des groupements comportant au moins une chaîne grasse, tels que les groupes alkyle, arylalkyle, alkylaryle comportant au moins 8 atomes de carbone ou des mélanges de ceux-ci.
      Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.
      On peut indiquer comme exemples d'alkylhydroxyéthylcelluloses quaternisées à chaînes grasses en C₈-C₃₀, les produits QUATRISOFT LM 200 ®, QUATRISOFT LM-X 529-18-A ®, QUATRISOFT LM-X 529-18B ® (alkyle en C₁₂) et QUATRISOFT LM-X 529-8 ® (alkyle en C₁₈) commercialisés par la société AMERCHOL et les produits CRODACEL QM ®, CRODACEL QL ® (alkyle en C₁₂) et CRODACEL QS ® (alkyle en C₁₈) commercialisés par la société CRODA.
- (III) Les polyvinyllactames cationiques dont la famille a été décrite par la demanderesse dans la demande de brevet français FR 0 101 106.
   Lesdits polymères comprennent :
   (a) au moins un monomère de type vinyl lactame ou alkylvinyllactame;
   (b) au moins un monomère de structures (IX) ou (X) suivantes : 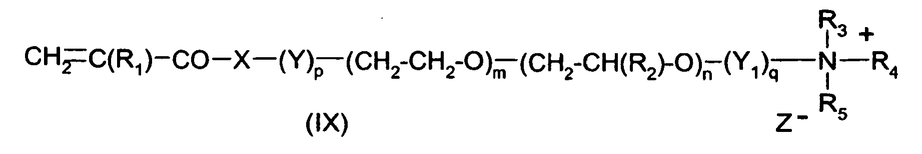 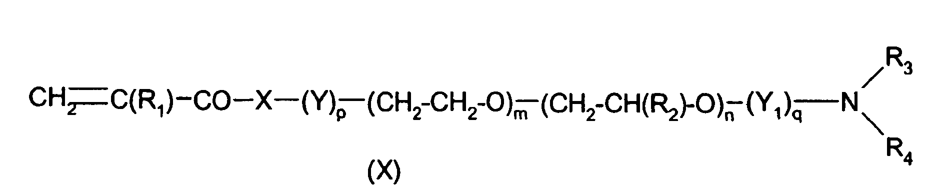 dans lesquelles :
      - X désigne un atome d'oxygène ou un radical NR₆ ;
      - R₁ et R₆ désignent, indépendamment l'un de l'autre, un atome d'hydrogène ou un radical alkyl linéaire ou ramifié en C₁-C₅ ;
      - R₂ désigne un radical alkyle linéaire ou ramifié en C₁-C₄;
      - R₃, R₄ et R₅ désignent, indépendamment l'un de l'autre, un atome d'hydrogène, un radical alkyle linéaire ou ramifié en C₁-C₃₀ ou un radical de formule (XI) :

         -(Y₂)ᵣ-(CH₂-CH(R₇)-O)ₓ-R₈ (XI)

         dans laquelle :
         - Y , Y₁ et Y₂ désignent, indépendamment l'un de l'autre, un radical alkylène linéaire ou ramifié en C₂-C₁₆ ;
         - R₇ désigne un atome d'hydrogène, ou un radical alkyle linéaire ou ramifié en C₁-C₄ ou un radical hydroxyalkyle linéaire ou ramifié en C₁-C₄ :
         - R₈ désigne un atome d'hydrogène ou un radical alkyle linéaire ou ramifié en C₁-C₃₀;
         - p, q et r désignent, indépendamment l'un de l'autre, soit la valeur zéro, soit la valeur 1 ;
         - m et n désignent, indépendamment l'un de l'autre, un nombre entier allant de 0 à 100 ;
         - x désigne un nombre entier allant de 1 à 100 ;
         - Z désigne un anion d'acide organique ou minéral ;
         sous réserve que :
         - l'un au moins des substituants R₃, R₄, R₅ ou R₈ désigne un radical alkyle linéaire ou ramifié en C₉-C₃₀;
         - si m ou n est différent de zéro, alors q est égal à 1;
         - si m ou n sont égaux à zéro, alors p ou q est égal à 0.

Les polymères poly(vinyllactame) cationiques selon l'invention peuvent être réticulés ou non réticulés et peuvent aussi être des polymères blocs.

De préférence, le contre ion Z⁻ des monomères de formule (1) est choisi parmi les ions halogénures, les ions phosphates, l'ion méthosulfate, l'ion tosylate.

De préférence, R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (IX) pour laquelle, encore plus préférentiellement, m et n sont égaux à zéro.

Le monomère vinyl lactame ou alkylvinyllactame est de préférence un composé de structure (XII) : 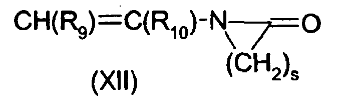 dans laquelle :
- s désigne un nombre entier allant de 3 à 6 ;
- R₉ désigne un atome d'hydrogène ou un radical alkyle en C₁-C₅ ;
- R₁₀ désigne un atome d'hydrogène ou un radical alkyle en C₁-C₅ ; sous réserve que l'un au moins des radicaux R₉ et R₁₀ désigne un atome d'hydrogène.

Encore plus préférentiellement, le monomère (XII) est la vinylpyrrolidone.

Les polymères poly(vinyllactame) cationiques selon l'invention peuvent également contenir un ou plusieurs monomères supplémentaires, de préférence cationiques ou non ioniques.

A titre de composés plus particulièrement préférés selon l'invention, on peut citer les terpolymères suivants comprenant au moins :
(a) un monomère de formule (XII) ;
(b) un monomère de formule (IX) dans laquelle p = 1, q = 0, R₃ et R₄ désignent, indépendamment l'un de l'autre, un atome d'hydrogène ou un radical alkyle en C₁-C₅ et R₅ désigne un radical alkyle en C₉-C₂₄ ; et
(c) un monomère de formule (X) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95 % de monomère (a), 0,1 à 55 % de monomère (c) et 0,25 à 50 % de monomère (b).

De tels polymères sont décrits dans la demande de brevet WO 00/68282 dont le contenu fait partie intégrante de l'invention.

Comme polymères poly(vinyllactame) cationiques selon l'invention, on utilise notamment les terpolymères vinylpyrrolidone / diméthylaminopropylméthacrylamide / tosylate de dodécyldiméthylméthacrylamidopropylammonium ; les terpolymères vinylpyrrolidone / diméthylaminopropylméthacrylamide / tosylate de cocoyldiméthylméthacrylamido propylammonium ; les terpolymères vinylpyrrolidone / diméthylaminopropyl méthacrylamide / tosylate ou chlorure de lauryldiméthylméthacrylamidopropylammonium.

La masse moléculaire en poids des polymères poly(vinyllactame) cationiques selon la présente invention est de préférence comprise entre 500 et 20 000 000. Elle est plus particulièrement comprise entre 200 000 et 2 000 000 et encore plus préférentiellement comprise entre 400 000 et 800 000.

Les polymères associatifs amphotères sont choisis de préférence parmi ceux comportant au moins un motif cationique non cyclique. Plus particulièrement encore, on préfère ceux préparés à partir ou comprenant 1 à 20 moles % de monomère comportant une chaîne grasse, et de préférence 1,5 à 15 moles % et plus particulièrement encore 1,5 à 6 moles %, par rapport au nombre total de moles de monomères.

Les polymères associatifs amphotères préférés selon l'invention comprennent, ou sont préparés en copolymérisant :
1) au moins un monomère de formule (XIII) ou (XIV): 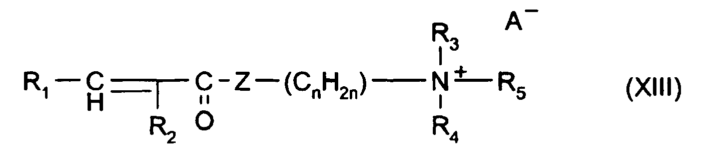 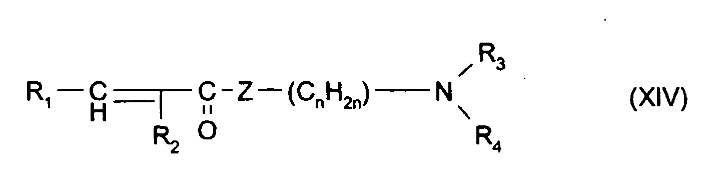 dans lesquelles :
   - R₁ et R₂, identiques ou différents, représentent un atome d'hydrogène ou un radical méthyle ;
   - R₃, R₄ et R₅, identiques ou différents, représente un radical alkyle linéaire ou ramifié ayant de 1 à 30 atomes de carbone ;
   - Z représente un groupe NH ou un atome d'oxygène ;
   - n est un nombre entier de 2 à 5 ;
   - A- est un anion issu d'un acide organique ou minéral, tel qu'un anion méthosulfate ou un halogénure tel que chlorure ou bromure ;
2) au moins un monomère de formule (XV)

   R₆-CH = CR₇-COOH (XV)

   dans laquelle :
   - R₆ et R₇, identiques ou différents, représentent un atome d'hydrogène ou un radical méthyle ; et
3) au moins un monomère de formule (XVI) :

   R₆-CH=CR₇-COXR₈ (XVI)

   dans laquelle :
   - R₆ et R₇, identiques ou différents, représentent un atome d'hydrogène ou un radical méthyle ;
   - X désigne un atome d'oxygène ou d'azote ; et
   - R₈ désigne un radical alkyle linéaire ou ramifié ayant de 1 à 30 atomes de carbone ;
l'un au moins des monomères de formule (XIII), (XIV) ou (XV) comportant au moins une chaîne grasse.

Les monomères de formule (XIII) et (XIV) de la présente invention sont choisis, de préférence, dans le groupe constitué par :
- le diméthylaminoéthylméthacrylate, le diméthylaminoéthylacrylate ;
- le diéthylaminoéthylméthacrylate, le diéthylaminoéthylacrylate ;
- le diméthylaminopropylméthacrylate, le diméthylaminopropylacrylate ;
- le diméthylaminopropylméthacrylamide, le diméthylaminopropylacrylamide ; ces monomères étant éventuellement quatemisés, par exemple par un halogénure d'alkyle en C₁-C₄ ou un sulfate de dialkyle en C₁-C₄.

Plus particulièrement, le monomère de formule (XIII) est choisi parmi le chlorure d'acrylamidopropyl triméthyl ammonium et le chlorure de méthacrylamidopropyl triméthyl ammonium.

Les monomères de formule (XIV) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (XIV) est l'acide acrylique.

Les monomères de formule (XIV) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et / ou quatemisés.

Le rapport du nombre de charges cationiques / charges anioniques est de préférence égal à environ 1.

Les polymères associatifs amphotères selon l'invention comprennent de préférence de 1 à 10 moles % du monomère comportant une chaîne grasse (monomère de formule (XIII), (XIV) ou (XV)), et de préférence de 1,5 à 6 moles %.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autre monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO 98/44012.

Parmi les polymères associatifs amphotères selon l'invention, on préfère les terpolymères acide acrylique / chlorure de (méth)acrylamidopropyl triméthyl ammonium / méthacrylate de stéaryle.

Selon l'invention, les polymères associatifs non ioniques sont choisis de préférence parmi :
- (1) les celluloses modifiées par des groupements comportant au moins une
   chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS ® (alkyles en C₁₆) vendu par la société AQUALON, ou le produit BERMOCOLL EHM 100 ® vendu par la société BEROL NOBEL ;
   - celles modifiées par des groupes polyalkylène glycol éther d'alkyl phénol, tel que le produit AMERCELL POLYMER HM-1500 ® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22 ® (chaîne alkyle en C₂₂) vendu par la société LAMBERTI, les produits RE210-18 ® (chaîne alkyle en C₁₄) et RE205-1 ® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216 ® ou GANEX V216 ® (copolymère vinylpyrrolidone / hexadécène) vendu par la société I.S.P. ;
   - les produits ANTARON V220 ® ou GANEX V220 ® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle / acrylate de stéaryle oxyéthyléné vendu par la société GOLDSCHMIDT sous la dénomination ANTIL 208 ®.
- (5) les copolymères de méthacrylates ou d'acrylates hydrophiles et de monomères hydrophobes comportant au moins une chaîne grasse tels que par exemple le copolymère méthacrylate de polyéthylèneglycol / méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et / ou des enchaînements cycloaliphatiques et / ou aromatiques.
- (7) les polymères à squelette aminoplaste éther possédant au moins une chaîne grasse, tels que les composés PURE THIX ® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse, ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205 ® à fonction urée vendu par la société RHEOX ou encore les Rhéolates ® 208 , 204 ou 212, ainsi que l'Acrysol RM 184 ®.

On peut également citer le produit ELFACOS T210 ® à chaîne alkyle en C₁₂₋₁₄ et le produit ELFACOS T212 ® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate ® 255, le Rhéolate ® 278 et le Rhéolate ® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46 ® et Aculyn 44 ® [l'ACULYN 46 ® est un polycondensat de polyéthylèneglycol à 150 ou 180 moles d'oxyde d'éthylène, d'alcool stéarylique et de méthylène bis(4-cyclohexyl-isocyanate) (SMDI), à 15 % en poids dans une matrice de maltodextrine (4 %) et d'eau (81 %) ; l'ACULYN 44 ® est un polycondensat de polyéthylèneglycol à 150 ou 180 moles d'oxyde d'éthylène, d'alcool décylique et de méthylène bis(4-cyclohexylisocyanate) (SMDI), à 35 % en poids dans un mélange de propylèneglycol (39 %) et d'eau (26 %)].

Dans une composition préférée selon la présente invention, les polymères associatifs sont choisis parmi les polymères associatifs non ioniques ou cationiques et plus particulièrement encore parmi les polyuréthanes polyéthers à séquences hydrophiles et hydrophobes, les polymères à squelette aminoplaste éther comportant au moins une chaîne grasse, les polyuréthanes associatifs cationiques, les dérivés de cellulose quaternisée comportant au moins une chaîne grasse, et les polyvinyllactames cationiques.

Dans une composition encore plus particulièrement préférée selon l'invention, le polymère associatif est choisi parmi les alkyl(C₈-C₃₀)hydroxyéthylcelluloses quaternisées et notamment la laurylhydroxyéthylcellulose quatemisée.

Les polymères associatifs non ioniques, anioniques, cationiques ou amphotères sont utilisés de préférence en une quantité pouvant varier d'environ 0,01 à 10 % en poids environ du poids total de la composition tinctoriale. Plus préférentiellement, cette quantité varie d'environ 0,1 à 5 % en poids, et encore plus particulièrement d'environ 0,5 à 3 % en poids.

La composition de la présente invention peut en outre comprendre une ou plusieurs bases d'oxydation additionnelles choisies parmi les bases d'oxydation classiquement utilisées en teinture d'oxydation différentes du para-aminophénol et du 3-méthyl 4-amino phénol. A titre d'exemple, ces bases d'oxydation additionnelles sont choisies parmi les para-phénylènediamines, les bis-phénylalkylènediamines, les para-aminophénols autres que le para-aminophénol et le 3-méthyl 4-amino phénol, les ortho-aminophénols, les bases hétérocycliques et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-(β-hydroxyéthyl) para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-(β-hydroxyéthyloxy) para-phénylènediamine, la 2-(β-acétytaminoéthytoxy) para-phénylènediamine, la N-(β-méthoxyéthyl) para-phénylènediamine, la 2-thiényl para-phénylènediamine, le 2-(β-hydroxyéthyl)amino 5-amino toluène et leurs sels d'addition.

Parmi les para-phénylènediamines citées ci-dessus, la para-phénylènediamine, la para-toluylènediamine, la 2-isopropyl para-phénylènediamine, la 2-(β-hydroxyéthyl) para-phénylènediamine, la 2-(β-hydroxyéthyloxy) para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-(β-acétylaminoéthyloxy) para-phénylènediamine et leurs sels d'addition sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4'-aminophényl) tétraméthylènediamine, la N,N'-bis-(β-hydroxyéthyl) N,N'-bis-(4'-aminophényl) tétraméthylènediamine, la N,N'-bis-(4'-méthyl-aminophényl) tétraméthylènediamine, la N,N'-bis-(éthyl) N,N'-bis-(4'-amino 3'-méthyl phényl) éthylènediamine, le 1,8-bis-(2,5-diamino phénoxy)-3,6-dioxaoctane et leurs sels d'addition.

Parmi les para-aminophénols autres que le para-aminophénol et le 3-méthyl 4-amino phénol, on peut citer à titre d'exemple, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 2-chloro 4-amino phénol, le 4-amino 6-[(5'-amino 2'-hydroxy 3'-méthyl phényl)méthyl] 2-méthyl phénol, le bis-(5-amino 2-hydroxy phényl) méthane, le 2,6-dichloro 4-amino phénol et leurs sels d'addition.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

De préférence, la composition de l'invention comprend comme uniques bases d'oxydation :
- au moins une première base d'oxydation choisie parmi le para-aminophénol et ses sels d'addition ; et
- au moins une deuxième base d'oxydation choisie parmi le 3-méthyl 4-amino phénol et ses sels d'addition.

Les bases d'oxydation présentes dans la composition de l'invention sont en général présentes chacune en quantité comprise entre 0,001 à 10 % en poids environ du poids total de la composition tinctoriale, de préférence entre 0,005 et 6 %.

La composition selon l'invention peut contenir en plus du 2-chloro 6-méthyl 3-amino phénol un ou plusieurs coupleurs additionnels choisis parmi les coupleurs conventionnellement utilisés pour la teinture d'oxydation des fibres kératiniques. Parmi ces coupleurs, on peut notamment citer les méta-phénylènediamines, les méta-aminophénols différents du 2-chloro 6-méthyl 3-amino phénol, les méta-diphénols, les coupleurs naphtaléniques, les coupleurs hétérocycliques ainsi que leur sels d'addition.

A titre d'exemple, on peut citer le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxy benzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxy pyridine, le 1-N-(β-hydroxyéthyl)amino-3,4-méthylènedioxy benzène, le 2,6-bis-(β-hydroxyéthylamino) toluène et leurs sels d'addition.

De préférence, la composition de l'invention comprend comme unique coupleur au moins un coupleur choisi parmi le 2-chloro 6-méthyl 3-amino phénol et ses sels d'addition.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre comprendre un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,001 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (1) suivante : 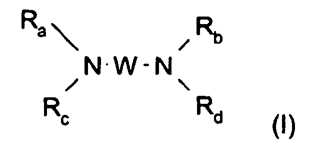 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel la composition conforme à l'invention telle que définie précédemment est appliquée sur les fibres kératiniques, et la couleur est révélée à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le comprenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale conforme à l'invention et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLE

Une composition tinctoriale est réalisée comme indiqué ci-dessous :

| **Exemple** | **1** |
|---|---|
| 2-chloro 6-méthyl 3-amino phénol | 1,37 g |
| Para-aminophénol | 0,475 g |
| 3-méthyl 4-amino phénol | 0,535 g |
| Support de teinture | (*) |
| Eau déminéralisée q.s.p. | 100 g |

| | |
|---|---|
| (*) : support de teinture commun | |

| | |
|---|---|
| Alcool décylique à 3 moles d'oxyde d'éthylène | 17,5 g |
| Alcool décylique à 5 moles d'oxyde d'éthylène | 4,5 g |
| Alcool laurique à 12 moles d'oxyde d'éthylène | 6,0 g |
| Alcool oléocétylique à 30 moles d'oxyde d'éthylène | 4,5 g |
| Acide oléique | 2,0 g |
| Alcool oléique | 1,8 g |
| Monoéthanolamide d'acide alkyl(C₁₃/C₁₅)éther carboxylique à 2 moles d'oxyde d'éthylène | 4,0 g |
| Glycérine | 3,0 g |
| Polycondensat tétraméthylhexaméthylène diamine / dichloro 1,3 propylène en solution aqueuse à 60 % | 2 g |
| Merquat 280 | 2,0 g |
| Agent séquestrant | q.s. |
| Réducteur | q.s. |
| Ammoniaque (20 % NH3) | 8,0 g |
| Aculyn 44 | 0,4 g |

Cette composition est mélangée, au moment de l'emploi, dans un bol en plastique et pendant 2 minutes, à une composition oxydante donnée ci-après, à raison de 1 partie de composition tinctoriale pour 1,5 partie de composition oxydante.

### Composition oxydante :

| | |
|---|---|
| Alcool cétylstéarylique | 2,3 g |
| Alcool cétylstéarylique à 30 moles d'oxyde d'éthylène | 0,6 g |
| Monoéthanolamide d'acide alkyl(C₁₃/C₁₅ 70/30 50 % linéaire) éther carboxylique à 2 moles d'oxyde d'éthylène | 0,9 g |
| Glycérine | 0,5 g |
| Peroxyde d'hydrogène | 7,5 g |
| Stabilisants | q.s. |
| Séquestrants | q.s. |
| Parfum | q.s. |
| Eau déminéralisée q.s.p. | 100 g |

On applique le mélange obtenu sur des mèches de cheveux naturels à 90 % de blancs et on laisse poser pendant 30 minutes.
On rince les mèches à l'eau, on les lave au shampooing standard, on les rince à l'eau à nouveau, puis on les sèche.
La coloration est évaluée de manière visuelle. Les nuances obtenues figurent dans le tableau ci-dessous.

| **Hauteur de ton** | **Reflet** |
|---|---|
| Blond clair | Rouge orangé |

## Revendications

1. Composition pour la teinture d'oxydation des fibres kératiniques comprenant, dans un milieu de teinture approprié :
- au moins un coupleur choisi parmi le 2-chloro 6-méthyl 3-amino phénol et ses sels d'addition ;
- au moins une première base d'oxydation choisie parmi le para-aminophénol et ses sels d'addition ;
- au moins une deuxième base d'oxydation choisie parmi le 3-méthyl 4-amino phénol et ses sels d'addition ; et
- au moins un polymère épaississant associatif.

2. Composition selon la revendication 1, dans laquelle les polymères épaississants associatifs comprennent des zones hydrophiles et des zones hydrophobes **caractérisées par** au moins une chaîne grasse en C₈-C₃₀.

3. Composition selon la revendication 1 ou 2, dans laquelle les polymères associatifs sont issus soit d'une polymérisation radicalaire ou d'une polycondensation à partir d'au moins un monomère dont l'un au moins possède une chaîne grasse en C₈-C₃₀, soit du greffage sur un polymère d'un composé comportant au moins une chaîne grasse en C₈-C₃₀.

4. Composition selon l'une quelconque des revendications précédentes, dans laquelle les polymères épaississants associatifs sont anioniques, cationiques, amphotères ou non ioniques.

5. Composition selon la revendication 4, dans laquelle au moins un des polymères associatifs est anionique.

6. Composition selon la revendication 5, dans laquelle les polymères associatifs anioniques comportent au moins un motif hydrophile de type acide carboxylique insaturé oléfinique et au moins un motif hydrophobe de type ester d'alkyl(C₁₀-C₃₀) d'acide carboxylique insaturé.

7. Composition selon la revendication 6, dans laquelle les polymères associatifs anioniques sont constitués d'acide acrylique, d'ester d'alkyl(C₁₂-C₂₂) d'acide acrylique ou métacrylique et d'agent réticulant.

8. Composition selon la revendication 7, dans laquelle les polymères associatifs anioniques sont constitués de 95 à 60 % en poids d'acide acrylique, de 4 à 40 % en poids d'acrylate d'alkyle en C₁₀-C₃₀ et de 0 à 6 % en poids de monomère insaturé polyéthylénique copolymérisable.

9. Composition selon la revendication 7, dans laquelle les polymères associatifs anioniques sont constitués de 98 à 96 % en poids d'acide acrylique, de 1 à 4 % en poids d'acrylate d'alkyle en C₁₀-C₃₀ et de 0,1 à 0,6 % en poids de monomère polymérisable réticulant.

10. Composition selon la revendication 5, dans laquelle les polymères associatifs anioniques sont des polyuréthanes.

11. Composition selon la revendication 4, dans laquelle au moins un des polymères associatifs est cationique.

12. Composition selon la revendication 11, dans laquelle les polymères associatifs cationiques sont des dérivés de cellulose quaternisée.

13. Composition selon la revendication 12, dans laquelle les dérivés de cellulose quaternisée sont des hydroxyéthylcelluloses quaternisées modifiées par des groupements comportant au moins une chaîne grasse.

14. Composition selon la revendication 13, dans laquelle la chaîne grasse des hydroxyéthylcelluloses est un radical alkyle comportant de 8 à 30 atomes de carbone.

15. Composition selon la revendication 11, dans laquelle les polymères associatifs cationiques sont des polyuréthanes.

16. Composition selon la revendication 4, dans laquelle au moins un des polymères associatifs est non ionique et choisi parmi les polyéthers polyuréthanes.

17. Composition selon la revendication 16, dans laquelle les polyéthers polyuréthanes sont des polycondensats de polyéthylèneglycol à 150 ou 180 moles d'oxyde d'éthylène, d'alcool stéarylique et d'au moins un diisocyanate.

18. Composition selon la revendication 17, dans laquelle les polyéthers polyuréthanes sont des polycondensats de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81 %).

19. Composition selon la revendication 16, dans laquelle les polyéthers polyuréthanes sont des polycondensats de polyéthylèneglycol à 150 ou 180 moles d'oxyde d'éthylène, d'alcool décylique et d'au moins un diisocyanate.

20. Composition selon la revendication 19, dans laquelle les polyéthers polyuréthanes sont des polycondensats de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%).

21. Composition selon l'une quelconque des revendications précédentes, comprenant au moins une base d'oxydation additionnelle choisie parmi les para-phénylènediamines, les bis-phénylalkylènediamines, les para-aminophénols autres que le para-aminophénol et le 3-méthyl 4-amino phénol, les ortho-aminophénols, les bases hétérocycliques ainsi que leurs sels d'addition.

22. Composition selon l'une quelconque des revendications 1 à 20, comprenant comme uniques bases d'oxydation :
- au moins une première base d'oxydation choisie parmi le para-aminophénol et ses sels d'addition ; et
- au moins une deuxième base d'oxydation choisie parmi le 3-méthyl 4-amino phénol et ses sels d'addition.

23. Composition selon l'une quelconque des revendications précédentes, comprenant au moins un coupleur additionnel choisi parmi les méta-phénylènediamines, les méta-aminophénols autres que le 2-chloro 6-méthyl 3-amino phénol, les méta-diphénols, les coupleurs naphtaléniques, les coupleurs hétérocycliques ainsi que leurs sels d'addition.

24. Composition selon l'une quelconque des revendications 1 à 22, comprenant comme unique coupleur au moins un coupleur choisi parmi le 2-chloro 6-méthyl 3-amino phénol et ses sels d'addition.

25. Composition selon l'une quelconque des revendications précédentes, dans laquelle la quantité de chacune des bases d'oxydation est comprise entre 0,001 à 10 % en poids environ du poids total de la composition tinctoriale.

26. Composition selon l'une quelconque des revendications précédentes, dans laquelle la quantité de chacun des coupleurs est comprise entre 0,001 et 10 % en poids environ du poids total de la composition tinctoriale.

27. Composition selon l'une quelconque des revendications 1 à 26, comprenant un agent oxydant.

28. Composition selon la revendication 27, dans laquelle l'agent oxydant est choisi parmi le peroxyde d'hydrogène, le peroxyde d'urée, les bromates de métaux alcalins, les persels, les peracides et les enzymes oxydases.

29. Procédé de teinture d'oxydation des fibres kératiniques, **caractérisé en ce qu'**une composition telle que définie à l'une quelconque des revendications 1 à 26 est appliquée sur les fibres kératiniques, et que la couleur est révélée à l'aide d'un agent oxydant.

30. Procédé selon la revendication 29, dans lequel l'agent oxydant est choisi parmi le peroxyde d'hydrogène, le peroxyde d'urée, les bromates de métaux alcalins, les persels, les peracides et les enzymes oxydases.

31. Procédé selon la revendication 29 ou 30, dans lequel l'agent oxydant est mélangé au moment de l'emploi à la composition telle que définie à l'une quelconque des revendications 1 à 26.

32. Procédé selon la revendication 29 ou 30, dans lequel une composition comprenant l'agent oxydant est appliquée sur les fibres kératiniques simultanément ou séquentiellement à la composition telle que définie à l'une quelconque des revendications 1 à 26.

33. Dispositif à plusieurs compartiments, dans lequel un premier compartiment contient une composition tinctoriale telle que définie à l'une quelconque des revendications 1 à 26 et un deuxième compartiment contient une composition comprenant un agent oxydant.

34. Utilisation de la composition tinctoriale telle que définie à l'une quelconque des revendications 1 à 28 pour la teinture d'oxydation des fibres kératiniques.

## Claims

1. Composition for the oxidation dyeing of keratin fibres, comprising, in a medium that is suitable for dyeing:
- at least one coupler chosen from 2-chloro-6-methyl-3-aminophenol and the addition salts thereof;
- at least one first oxidation base chosen from para-aminophenol and the addition salts thereof;
- at least one second oxidation base chosen from 3-methyl-4-aminophenol and the addition salts thereof; and
- at least one associative thickening polymer.

2. Composition according to Claim 1, in which the associative thickening polymers comprise hydrophilic regions and hydrophobic regions **characterized by** at least one C₈-C₃₀ fatty chain.

3. Composition according to Claim 1 or 2, in which the associative polymers are derived either from a free-radical polymerization or a polycondensation starting with at least one monomer, at least one of which contains a C₈-C₃₀ fatty chain, or from the grafting of a compound comprising at least one C₈-C₃₀ fatty chain onto a polymer.

4. Composition according to any one of the preceding claims, in which the associative thickening polymers are anionic, cationic, amphoteric or nonionic.

5. Composition according to Claim 4, in which at least one of the associative polymers is anionic.

6. Composition according to Claim 5, in which the anionic associative polymers comprise at least one hydrophilic unit of olefinic unsaturated carboxylic acid type and at least one hydrophobic unit of (C₁₀-C₃₀)alkyl ester of unsaturated carboxylic acid type.

7. Composition according to Claim 6, in which the anionic associative polymers consist of acrylic acid, an acrylic or methacrylic acid (C₁₂-C₂₂)alkyl ester and a crosslinking agent.

8. Composition according to Claim 7, in which the anionic associative polymers consist of from 95% to 60% by weight of acrylic acid, from 4% to 40% by weight of a C₁₀-C₃₀ alkyl acrylate and from 0% to 6% by weight of copolymerizable polyethylenic unsaturated monomer.

9. Composition according to Claim 7, in which the anionic associative polymers consist of from 98% to 96% by weight of acrylic acid, from 1% to 4% by weight of a C₁₀-C₃₀ alkyl acrylate and from 0.1% to 0.6% by weight of crosslinking polymerizable monomer.

10. Composition according to Claim 5, in which the anionic associative polymers are polyurethanes.

11. Composition according to Claim 4, in which at least one of the associative polymers is cationic.

12. Composition according to Claim 11, in which the cationic associative polymers are quaternized cellulose derivatives.

13. Composition according to Claim 12, in which the quaternized cellulose derivatives are quaternized hydroxyethylcelluloses modified with groups comprising at least one fatty chain.

14. Composition according to Claim 13, in which the fatty chain of the hydroxyethylcelluloses is an alkyl radical containing from 8 to 30 carbon atoms.

15. Composition according to Claim 11, in which the cationic associative polymers are polyurethanes.

16. Composition according to Claim 4, in which at least one of the associative polymers is nonionic and chosen from polyurethane polyethers.

17. Composition according to Claim 16, in which the polyurethane polyethers are polycondensates of polyethylene glycol containing 150 or 180 mol of ethylene oxide, of stearyl alcohol and of at least one diisocyanate.

18. Composition according to Claim 17, in which the polyurethane polyethers are polycondensates of polyethylene glycol containing 150 or 180 mol of ethylene oxide, of stearyl alcohol and of methylenebis(4-cyclohexyl isocyanate) (SMDI), at a concentration of 15% by weight in a matrix of maltodextrin (4%) and of water (81%).

19. Composition according to Claim 16, in which the polyurethane polyethers are polycondensates of polyethylene glycol containing 150 or 180 mol of ethylene oxide, of decyl alcohol and of at least one diisocyanate.

20. Composition according to Claim 19, in which the polyurethane polyethers are polycondensates of polyethylene glycol containing 150 or 180 mol of ethylene oxide, of decyl alcohol and of methylenebis(4-cyclohexyl isocyanate) (SMDI), at a concentration of 35% by weight in a mixture of propylene glycol (39%) and water (26%).

21. Composition according to any one of the preceding claims, comprising at least one additional oxidation base chosen from para-phenylenediamines, bisphenylalkylenediamines, para-aminophenols other than para-aminophenol and 3-methyl-4-aminophenol, ortho-aminophenols and heterocyclic bases, and the addition salts thereof.

22. Composition according to any one of Claims 1 to 20, comprising as sole oxidation bases:
- at least one first oxidation base chosen from para-aminophenol and the addition salts thereof; and
- at least one second oxidation base chosen from 3-methyl-4-aminophenol and the addition salts thereof.

23. Composition according to any one of the preceding claims, comprising at least one additional coupler chosen from meta-phenylenediamines, meta-aminophenols other than 2-chloro-6-methyl-3-aminophenol, meta-diphenols, naphthalene-based couplers and heterocyclic couplers, and the addition salts thereof.

24. Composition according to any one of Claims 1 to 22, comprising as sole coupler at least one coupler chosen from 2-chloro-6-methyl-3-aminophenol and the addition salts thereof.

25. Composition according to any one of the preceding claims, in which the amount of each of the oxidation bases is between 0.001% and 10% by weight approximately relative to the total weight of the dye composition.

26. Composition according to any one of the preceding claims, in which the amount of each of the couplers is between 0.001% and 10% by weight approximately relative to the total weight of the dye composition.

27. Composition according to any one of Claims 1 to 26, comprising an oxidizing agent.

28. Composition according to Claim 27, in which the oxidizing agent is chosen from hydrogen peroxide, urea peroxide, alkali metal bromates, persalts, peracids and oxidase enzymes.

29. Process for the oxidation dyeing of keratin fibres, **characterized in that** a composition as defined in any one of Claims 1 to 26 is applied to the keratin fibres, and the colour is developed using an oxidizing agent.

30. Process according to Claim 29, in which the oxidizing agent is chosen from hydrogen peroxide, urea peroxide, alkali metal bromates, persalts, peracids and oxidase enzymes.

31. Process according to Claim 29 or 30, in which the oxidizing agent is mixed at the time of use with the composition as defined in any one of Claims 1 to 26.

32. Process according to Claim 29 or 30, in which a composition comprising the oxidizing agent is applied to the keratin fibres simultaneously with or sequentially to the composition as defined in any one of Claims 1 to 26.

33. Multi-compartment device, in which a first compartment contains a dye composition as defined in any one of Claims 1 to 26 and a second compartment contains a composition comprising an oxidizing agent.

34. Use of the dye composition as defined in any one of Claims 1 to 28, for the oxidation dyeing of keratin fibres.

## Patentansprüche

1. Zusammensetzung zum oxidativen Färben von Keratinfasern, die in einem zum Färben geeigneten Medium enthält:
- mindestens einen Kuppler, der unter 2-Chlor-6-methyl-3-amino-phenol und seinen Additionssalzen ausgewählt ist,
- mindestens eine erste Oxidationsbase, die unter p-Aminophenol und seinen Additionssalzen ausgewählt ist,
- mindestens eine zweite Oxidationsbase, die unter 3-Methyl-4-amino-phenol und seinen Additionssalzen ausgewählt ist, und
- mindestens ein assoziatives verdickendes Polymer.

2. Zusammensetzung nach Anspruch 1, wobei die verdickenden assoziativen Polymere hydrophile Bereiche und hydrophobe Bereiche aufweisen, die durch mindestens eine C₈₋₃₀-Fettkette **gekennzeichnet** sind.

3. Zusammensetzung nach Anspruch 1 oder 2, wobei die assoziativen Polymere entweder durch radikalische Polymerisation oder Polykondensation ausgehend von mindestens einem Monomer, von denen mindestens eines eine Fettkette mit 8 bis 30 Kohlenstoffatomen besitzt, oder durch Pfropfen einer Verbindung, die mindestens eine Fettkette mit 8 bis 30 Kohlenstoffatomen aufweist, auf ein Polymer gebildet werden.

4. Zusammensetzung nach einem der vorhergehenden Ansprüche, wobei die verdickenden assoziativen Polymere anionisch, kationisch, amphoter oder nichtionisch sind.

5. Zusammensetzung nach Anspruch 4, wobei mindestens ein assoziatives Polymer anionisch ist.

6. Zusammensetzung nach Anspruch 5, wobei die anionischen assoziativen Polymere mindestens eine hydrophile Einheit vom Typ einer olefinisch ungesättigten Carbonsäure und mindestens eine hydrophobe Einheit vom Typ eines Alkyl(C₁₀₋₃₀)esters einer ungesättigten Carbonsäure enthalten.

7. Zusammensetzung nach Anspruch 6, wobei die anionischen assoziativen Polymere aus Acrylsäure, einem Alkyl(C₁₂₋₂₂)acrylsäureester oder Alkyl(C₁₂₋₂₂)methacrylsäureester und einem Vernetzungsmittel bestehen.

8. Zusammensetzung nach Anspruch 7, wobei die anionischen assoziativen Polymere aus 95 bis 60 Gew.-% Acrylsäure, 4 bis 40 Gew.-% C₁₀₋₃₀-Alkylacrylat und 0 bis 6 Gew.-% eines copolymerisierbaren, mehrfach ethylenisch ungesättigten Monomers bestehen.

9. Zusammensetzung nach Anspruch 7, wobei die anionischen assoziativen Polymere aus 98 bis 96 Gew.-% Acrylsäure, 1 bis 4 Gew.-% C₁₀₋₃₀-Alkylacrylat und 0,1 bis 0,6 Gew.-% eines polymerisierbaren vernetzenden Monomers bestehen.

10. Zusammensetzung nach Anspruch 5, wobei die anionischen assoziativen Polymere Polyurethane sind.

11. Zusammensetzung nach Anspruch 4, wobei mindestens ein assoziatives Polymer kationisch ist.

12. Zusammensetzung nach Anspruch 11, wobei die kationischen assoziativen Polymere Derivate von quaternisierter Cellulose sind.

13. Zusammensetzung nach Anspruch 12, wobei die Derivate von quaternisierter Cellulose quaternisierte Hydroxyethylcellulosen sind, die mit Gruppen modifiziert sind, die mindestens eine Fettkette aufweisen.

14. Zusammensetzung nach Anspruch 13, wobei die Fettkette der Hydroxyethylcellulosen eine Alkylgruppe mit 8 bis 30 Kohlenstoffatomen ist.

15. Zusammensetzung nach Anspruch 11, wobei die kationischen assoziativen Polymere Polyurethane sind.

16. Zusammensetzung nach Anspruch 4, wobei mindestens ein assoziatives Polymer nichtionisch ist und unter den Polyetherpolyurethanen ausgewählt ist.

17. Zusammensetzung nach Anspruch 16, wobei die Polyetherpolyurethane Polykondensate von Polyethylenglycol mit 150 bis 180 mol Ethylenoxid, Stearylalkohol und mindestens einem Diisocyanat sind.

18. Zusammensetzung nach Anspruch 17, wobei die Polyetherpolyurethane Polykondensate von Polyethylenglycol mit 150 bis 180 mol Ethylenoxid, Stearylalkohol und Methylen-bis(4-cyclohexylisocyanat) (SMDI) von 15 Gew.-% in einer Matrix aus Maltodextrin (4 %) und Wasser (81 %) sind.

19. Zusammensetzung nach Anspruch 16, wobei die Polyetherpolyurethane Polykondensate von Polyethylenglycol mit 150 bis 180 mol Ethylenoxid, Decylalkohol und mindestens einem Diisocyanat sind.

20. Zusammensetzung nach Anspruch 19, wobei die Polyetherpolyurethane Polykondensate von Polyethylenglycol mit 150 bis 180 mol Ethylenoxid, Decylalkohol und Methylen-bis(4-cyclohexylisocyanat) (SMDI) von 35 Gew.-% in einem Gemisch aus propylenglycol (39 %) und Wasser (26 %) sind.

21. Zusammensetzung nach einem der vorhergehenden Ansprüche, die mindestens eine ergänzenden Oxidationsbase enthält, die unter den p-Phenylendiaminen, Bisphenylalkylendiaminen, p-Aminophenolen, die von p-Aminophenol und 3-Methyl-4-aminophenol verschieden sind, o-Aminophenolen und den heterocyclischen Basen und deren Additionssalzen ausgewählt sind.

22. Zusammensetzung nach einem der Ansprüche 1 bis 20, die als einzige Oxidationsbasen enthält:
- mindestens eine erste Oxidationsbase, die unter p-Aminophenol und seinen Additionssalzen ausgewählt ist; und
- mindestens eine zweite Oxidationsbase, die unter 3-Methyl-4-aminophenol und seinen Additionssalzen ausgewählt ist.

23. Zusammensetzung nach einem der vorhergehenden Ansprüche, die mindestens einen ergänzenden Kuppler enthält, der unter den *m*-Phenylendiaminen, *m*-Aminophenolen, die von dem 2-Chlor-6-methyl-3-amino-phenol verschieden sind, *m*-Dihydroxybenzolen, Naphthalinkupplern und heterocyclischen Kupplern sowie deren Additionssalzen ausgewählt ist.

24. Zusammensetzung nach einem der Ansprüche 1 bis 22, die als einzigen Kuppler mindestens einen Kuppler enthält, der unter 2-Chlor-6-methyl-3-amino-phenol und seinen Additionssalzen ausgewählt ist.

25. Zusammensetzung nach einem der vorhergehenden Ansprüche, wobei der Mengenanteil jeder Oxidationsbase im Bereich von etwa 0,001 bis 10 Gew.-% des Gesamtgewichts der Zusammensetzung liegt.

26. Zusammensetzung nach einem der vorhergehenden Ansprüche, wobei der Mengenanteil jedes Kupplers im Bereich von etwa 0,001 bis 10 Gew.-% des Gesamtgewichts der Zusammensetzung liegt.

27. Zusammensetzung nach einem der Ansprüche 1 bis 26, die ein Oxidationsmittel enthält.

28. Zusammensetzung nach Anspruch 27, wobei das Oxidationsmittel unter Wasserstoffperoxid, Harnstoffperoxid, Alkalimetallbromaten, Salzen von Persäuren, Persäuren und Oxidasen ausgewählt ist.

29. Verfahren zum oxidativen Färben von Keratinfasern, **dadurch gekennzeichnet, dass** auf die Fasern mindestens eine Zusammensetzung nach einem der Ansprüche 1 bis 26 aufgetragen und die Farbe mit einem Oxidationsmittel gebildet wird.

30. Verfahren nach Anspruch 29, wobei das Oxidationsmittel unter Wasserstoffperoxid, Harnstoffperoxid, Alkalimetallbromaten, Salzen von Persäuren, Persäuren und Oxidasen ausgewählt ist.

31. Verfahren nach Anspruch 29 oder 30, wobei das Oxidationsmittel bei der Anwendung mit der Zusammensetzung nach einem der Ansprüche 1 bis 26 vermischt wird.

32. Verfahren nach Anspruch 29 oder 30, wobei eine Zusammensetzung, die das Oxidationsmittel enthält, gleichzeitig mit oder nach der Zusammensetzung nach einem der Ansprüche 1 bis 26 auf die Keratinfasern aufgetragen wird.

33. Vorrichtung mit mehreren Abteilungen, wobei eine erste Abteilung eine Farbmittelzusammensetzung nach einem der Ansprüche 1 bis 26 enthält, und eine zweite Zusammensetzung eine Zusammensetzung mit einem Oxidationsmittel enthält.

34. Verwendung der Farbmittelzusammensetzung nach einem der Ansprüche 1 bis 28 zum oxidativen Färben von Keratinfasern.
